# EUROPEAN PATENT APPLICATION

(11) **EP 1 944 288 A1**
(43) Date of publication of application: **16.07.2008**
(21) Application number: 07090001.4
(22) Date of filing: 09.01.2007
(51) Int. Cl.: C07C 311/19, C07D 203/08, C07D 203/24, C07D 207/14, C07D 403/12, C07D 405/04, C07D 405/14, C07D 487/04, C07D 487/22, C07D 491/04, C07K 7/02

(54) **Radiolabelling via fluorination of aziridines**

(71) Applicant: Bayer Schering Pharma Aktiengesellschaft, 13353 Berlin (DE)
(72) Inventor: Voigtmann, Ulrike, 10115 Berlin (DE); Srinivasan, Ananth, 10629 Berlin (DE); Stellfeld, Timo, 14197 Berlin (DE); Brumby, Thomas, 12163 Berlin (DE); Graham, Keith, 10115 Berlin (DE); Becaud, Jessica, 4600 Olten (CH)

(57) **Abstract**

This invention relates to novel compounds comprising arziridine ring suitable for labelling or already labelled with an appropriate halogen, preferably ¹⁸F, methods of preparing such compounds, compositions comprising such compounds, kits comprising such compounds or compositions and uses of such compounds, compositions or kits for diagnostic imaging, preferably positron emission tomography (PET).

## Description

### Field of Invention

This invention relates to novel compounds suitable for labelling or already labelled with an appropriate halogen, preferably ¹⁸F, methods of preparing such compounds, compositions comprising such compounds, kits comprising such compounds or compositions and uses of such compounds, compositions or kits for diagnostic imaging, preferably positron emission tomography (PET).

### Background Art

Molecular imaging has the potential to detect disease progression or therapeutic effectiveness earlier than most conventional methods in the fields of oncology, neurology and cardiology. Of the several promising molecular imaging technologies having been developed as optical imaging and MRI, PET is of particular interest for drug development because of its high sensitivity and ability to provide quantitative and kinetic data.

Positron emitting isotopes include carbon, nitrogen, and oxygen. These isotopes can replace their non-radioactive counterparts in target compounds to produce tracers that function biologically and are chemically identical to the original molecules for PET imaging. On the other hand, ¹⁸F is the most convenient labelling isotope due to its relatively long half life (109.6 min) which permits the preparation of diagnostic tracers and subsequent study of biochemical processes. In addition, its low β+ energy (635 keV) is also advantageous.

PET tracers are or often include a molecule of biological interest. Biomolecules developed for use in PET have been numerous intended for specific targeting in the patient as e.g. FDG, FLT, L-DOPA, methionine and deoxythymidine. Due to their specific use, such biomolecule are often designated as "targeting agents".

Several approaches for incorporating ¹⁸F in more complex biomolecules as e.g. peptides are described in the following references: European J Nucl. Med. Mol. Imaging 2001, 28, 929-938; European J Nucl. Med. Mol. Imaging 2004, 31, 1182-1206; Bioconjugate Chem. 1991, 2, 44-49; Bioconjugate Chem. 2003, 14, 1253-1259.

These methods are indirect. They demand at least a two step procedure for tracer synthesis. Therefore they are time consuming thereby reducing PET image resolution as a result of nuclear decay.

Object of the present invention is therefore the development of a practical and mild technique for radio-labelling, in particular ¹⁸F labelling, of complex biomolecules like peptides in only one rather than two or more chemical steps in order to save time, costs and additional purification steps of radioactive compounds.

### Summary of the Invention

The object of the present invention is solved as detailed below.

In the first place, the present invention provides novel compounds comprising an for labelling purposes appropriately activated aziridine ring, wherein a targeting agent radical, either directly or via an appropriate linker, is attached either to the aziridine ring or to a fused 5-member carbocyclic or heterocyclic ring.

In one alternative, such compound is represented by formula I 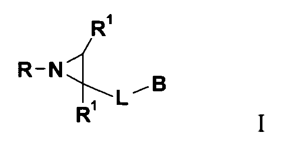 wherein
R represents Ts, 2,4,6-triisopropyl-phenyl-sulfonyl, 3,4-dimethoxy-phenyl-sulfonyl, phenyl-sulfonyl, with 1-5 R² substituted phenyl-sulfonyl, Ns, Cbz, Bz, Bn, Boc, Fmoc, methoxycarbonyl, ethoxycarbonyl, allyloxycarbonyl, Tr, or acyl;
R¹ represents hydrogen, substituted or non-substituted, linear or branched C₁-C₆ alkyl, cycloalkyl, heterocycloalkyl, aryl, heteroaryl, aralkyl, or heteroaralkyl;
R² represents hydrogen, substituted or non-substituted, linear or branched C₁-C₆ alkyl, cycloalkyl, heterocycloalkyl, aryl, heteroaryl, aralkyl, or heteroaralkyl, OH, OR³, NH₂, NHR³, NR³₂, SH, SR³, halogens, nitro, COR³, COOR³, CONHR³, CONR³₂;
R³ represents hydrogen, substituted or non-substituted, linear or branched C₁-C₆ alkyl, aryl, cycloalkyl, heterocycloalkyl, aryl, heteroaryl, aralkyl, or heteroaralkyl;
L represents a linker suitable for coupling with the targeting agent radical; and
B represents the targeting agent radical.

In another alternative, the compound is represented by formula II 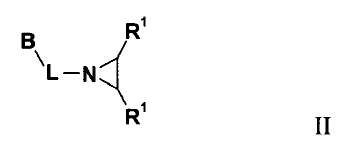 wherein
R¹, independently represents hydrogen, substituted or non-substituted, linear or branched C₁-C₆ alkyl, cycloalkyl, heterocycloalkyl, aryl, heteroaryl, aralkyl, or heteroaralkyl;
L represents a linker suitable for coupling with the targeting agent radical and appropriate activation of the aziridine; and
B represents the targeting agent radical.

In a third alternative, the compound is represented by formula III 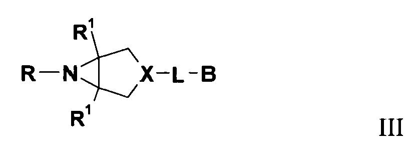 wherein
R represents Ts, 2,4,6-triisopropyl-phenyl-sulfonyl, 3,4-dimethoxy-phenyl-sulfonyl, phenyl-sulfonyl, with 1-5 R² substituted phenyl-sulfonyl, Ns, Cbz, Bz, Bn, Boc, Fmoc, methoxycarbonyl, ethoxycarbonyl, allyloxycarbonyl, Tr, or acyl;
R¹ represents hydrogen, substituted or non-substituted, linear or branched C₁-C₆ alkyl, cycloalkyl, heterocycloalkyl, aryl, heteroaryl, aralkyl, or heteroaralkyl;
R² represents hydrogen, substituted or non-substituted, linear or branched C₁-C₆ alkyl, cycloalkyl, heterocycloalkyl, aryl, heteroaryl, aralkyl, or heteroaralkyl, OH, OR³, NH₂, NHR³, NR³₂, SH, SR³, halogens, nitro, COR³, COOR³, CONHR³, CONR³₂;
R³ represents hydrogen, substituted or non-substituted, linear or branched C₁-C₆ alkyl, aryl, cycloalkyl, heterocycloalkyl, aryl, heteroaryl, aralkyl, or heteroaralkyl;
X represents N or C substituted by a hydrogen;
L represents a linker suitable for coupling with the targeting agent radical; and
B represents the targeting agent radical.

In all cases, the linker L is preferably selected from the group consisting of substituted or non-substituted, linear or branched C₁-C₆ alkyl, cycloalkyl, alkenyl, heterocycloalkyl, aryl, heteroaryl, substituted aryl or heteroaryl, aralkyl, heteroaralkyl, alkoxy, aryloxy, aralkoxy, directly bound to an alkyl chain or aryl or -CO-, -C(=O)O-, -C(=O)NH-, -SO₂-, -SO₂NR³-, -NR³SO₂-, -NR³C(=O)O-, -NR³C(=O)NR³-, -NR³-, -NH-NH-, -NH-O- 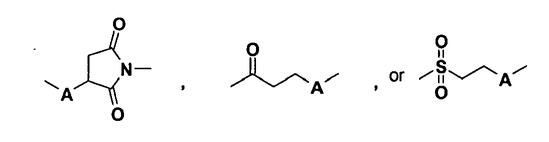
wherein A represents S or N; and
R³ represents hydrogen substituted or non-substituted, linear or branched C₁-C₆ alkyl, cycloalkyl, heterocycloalkyl, aryl, heteroaryl, aralkyl or heteroaralkyl.

Preferably, the targeting agent is selected from peptides, small molecules or oligonucleotides.

More preferably, the targeting agent is -NR⁴-peptide, R⁴ being independently selected from hydrogen or alkyl, or -(CH₂)ₙ-peptide and wherein n is between 1 and 6.

Most preferably, the targeting agent is a peptide comprising from 4 to 100 amino acids.

A further aspect of this invention is directed to a method of preparing the novel compounds by reacting a suitable precursor molecule with the targeting agent or a precursor thereof.

Another aspect of the present invention relates to a compound obtainable by a ring opening halogenation reaction of the aziridine ring of one of the novel compounds, or a pharmaceutically acceptable salt or solvate thereof.

Preferably, the halogen introduced is ¹⁸F, ¹⁹F, ⁷⁵Br, ⁷⁶Br, ¹²³I, ¹³¹I or ^{34m}Cl, most preferably ¹⁸ F.

Furthermore, the present invention is directed to a method of preparing such compounds by reacting one of the novel compounds defined hereinabove with an appropriate halogenation agent under appropriate reaction conditions.

Preferably, said halogenation agent is K¹⁸F, H¹⁸F, KH¹⁸F₂ or the tetraalkyl ammonium salt of ¹⁸F⁻.

The method is preferably run under a reaction temperature of 100°C or less, most preferably 80°C or less.

The present invention is also directed to a composition comprising a halogenated compound as defined hereinabove, preferably together with the physiologically acceptable diluent or carrier.

Moreover, the present invention is directed to a kit comprising any of the halogenated compounds as defined hereinabove or a composition comprising the same, in powder form, and a container containing an appropriate solvent for preparing a solution of the compound or composition for administration to an animal, including a human.

Finally, the present invention is directed to the use of any halogenated compound, as defined hereinabove, or respective composition or kit, for diagnostic imaging, in particular positron emission tomography.

Most preferably for imaging of tumors, imaging of inflammatory and/or neurodegenerative diseases, such as multiple sclerosis of Alzheimer's disease, or imaging of angiogenesis-associates diseases, such as growth of solid tumors, and rheumatoid arthritis.

Embodiments of this invention include methods involving the ¹⁸F fluorination of compounds ready for use as imaging agents and the ¹⁸F containing compounds derived from them. The compounds subjected to fluorination may already include a targeting agent for imaging purposes. Preferred embodiments of this invention involve the formation of a precursor molecule, which may include a targeting agent, prior to fluorination with ¹⁸F being the last step in the process prior to preparation of the compound for administration to an animal, in particular a human.

The use of aziridines described herein facilitates the process. Thus, a desired PET imaging agent is proposed starting from an aziridine which is then subjected to ¹⁸F fluorination.

Substituents on such aziridines include linking groups or reactive groups designed for subsequent addition of a targeting agent. Linking groups may include aliphatic or aromatic molecules and readily form a bond to a selected, appropriate functionalized targeting agent. A variety of such groups is known in the art. These include carboxylic acids, carboxylic acid chlorides and active esters, sulfonic acids, sulfonyl-chlorides, amines, hydroxides, thiols etc. on either side.

Contemplated herein are also groups which provide for ionic, hydrophobic and other non-convalent bonds between the aziridine ring and the targeting agent.

Very few publications are known which describe the opening of aziridines by ¹⁸F: L. Tron et al., describe the reaction of an aziridine moiety with 18F- in the synthesis of [18F]FNECA. The desired product, however, was obtained in very low yields only. (Journal of Labelled Compounds and Radiopharmaceuticals 2000, 43, 807-815.)

W. Feindel et al., synthesized [18F]BFNU and [18F]CFNU by nucleophilic attack of 18F-TBAF on the aziridine ring of 1,3-substituted ureas in low yields. (Canadian Journal Chemistry 1984, 62, 2107-2112).

These mentioned ¹⁸F-labelled aziridine precursors cannot be coupled to chemical functionalities like amines, thiols, hydroxyls, carboxylic acid functions or other chemical groups of complex targeting agents without further transformations.

Even publications about cold fluorinations are at a manageable quantity and rather performed with
BF3·OEt2: Synlett 2004, 12, 2218-2220.; Recl. Trav. Chim. Pays-Bas 1992, 111(2), 59-68.;
HF*Pyridine (Olah's Reagent): Journal of Chemical Research, Synopses 1983, 10, 246-7*.;* Journal of the Chemical Society, Perkin Transactions 1: Organic and Bio-Organic Chemistry 1983, 9, 2045-51.; Journal of Organic Chemistry 1981, 46(24), 4938-48.; Journal of Fluorine Chemistry 1980, 16(3), 277-83.; Journal of Fluorine Chemistry 1980, 16(2), 183-7.; Journal of Organic Chemistry 1980, 45(26), 5328-33.; Tetrahedron Letters 1980, 21(3), 289-92.; Journal of Fluorine Chemistry 1990, 49(2), 231-46.; Tetrahedron 1987, 43(11), 2485-92.; Tetrahedron Letters 1978, 35, 3247-50.; Journal of Fluorine Chemistry 1981, 18, 93-96.; Journal of Fluorine Chemistry 1980, 16, 277-84.; Journal of Medicinal Chemistry 1990, 33(9), 2603-2610.; Journal of Fluorine Chemistry 1980, 16, 538-539.;
Pyridiniumpolyhydrogenfluoride: Journal of Fluorine Chemistry 1983, 23, 481.;
DAST: Tetrahedron 1999, 55(48), 13819-13830*.;* or
LiBF4: Journal of Organic Chemistry 1989, 54(22), 5324-30*.,*
than with nucleophilic fluorination reagents preferred in ¹⁸F fluorinations such as
TBAF: Carbohydrate Research 2003, 338(24), 2825-34.; Journal of Organic Chemistry 1989, 54(22), 5324-5330.; Carbohydrate Research 1980, 83, 142-145.; Tetrahedron Asymmetry 2004, 15(20), 3307-3322.;
KHF2: Carbohydrate Research 1992, 230, 89-106.; or
KF: Journal of Organic Chemistry 2004, 69(2), 335-338.

Preparation of ¹⁸F-labelled 2-fluoroethylamines, -amides and -sulfonamides is normally performed by at least two step procedures applying ¹⁸F-2-fluorethylamine or 2-bromofluorethane. Opening of appropriate aziridines may deliver such structural motifs by single step synthesis.

Peptides containing aziridines are described in several publications but the purpose of their synthesis, their substitution pattern and their applications are different from the use as precursor for radioactive labelling claimed herein.

A method for site- and stereoselective peptide modification using aziridine-2-carboxylic acid-containing peptides for site-selective conjugation with various thiol nucleophiles is described.

Journal of the American Chemical Society 2005, 127(20), 7359-7369. Journal of the American Chemical Society 2004, 126(40), 12712-12713.

A ligand with an aziridine-containing side chain designed to mimic arginine and to bind covalently in the arginine-specific P2 pocket of the class I major histocompatibility complex (MHC) glycoprotein HLA-B27 has been synthesized which alkylates specifically cysteine 67. Proceedings of the National Academy of Sciences of the United States of America 1996, 93(20), 10945-10948.

An aziridine containing lysine derivative as a potential LSD 1 inhibitor based on structural considerations and in analogy to known strategies for blocking amine oxidases has been prepared. Journal of the American Chemical Society 2006, 128(14), 4536-4537.

Small molecules with aziridines modified peptides have been claimed as antidepressant compounds to treat patients suffering from depression. WO 9922758

The preparation of reactive peptide ligands containing aziridines used to change the kinetics of binding by reacting with the protein when bound thereby forming covalent peptide ligand-protein complexes has been claimed. WO 9814208

As used herein, the term "alkyl", by itself or as part of another group, refers to a straight chain or branched chain alkyl group with 1 to 20 carbon atoms such as, for example methyl, ethyl, propyl, isopropyl, butyl, isobutyl, tert-butyl, pentyl, isopentyl, neopentyl, heptyl, hexyl, and decyl. Alkyl groups can also be substituted, such as by halogen atoms, hydroxyl groups, C₁-C₄-alkoxy groups or C₆-C₁₂-aryl groups (which, in turn, can also be substituted, such as by 1 to 3 halogen atoms).

"Alkenyl" and "alkinyl" are similarly defined, but contain at least one carbon-carbon double or triple bond, respectively.

As used herein, the term "aryl", by itself or as part of another group, refers to monocyclic or bicyclic aromatic groups containing from 6 to 12 carbons in the ring portion, preferably 6-10 carbons in the ring portion, such as phenyl, naphthyl or tetrahydronaphthyl.

As used herein, the term "heteroaryl", by itself or as part of another group, refers to groups having 5 to 14 ring atoms; 6, 10 or 14 II electrons shared in a cyclic array; and containing carbon atoms and 1, 2, 3 or 4 oxygen, nitrogen or sulfur heteroatoms.

Examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxythiinyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, 4aH-carbazolyl, carbazolyl, carbolinyl, phenanthridinyl, acridinyl, perimidinyl, phenanthrolinyl, phenazinyl, isothiazolyl, phenothiazinyl, isoxazolyl, furazanyl und phenoxazinyl.

As used herein, the term "targeting agent" is directed to a compound or moiety that targets or directs the radionuclide attached to it to a specific site in a biological system. A targeting agent can be any compound or chemical entity that binds to or accumulates at a target site in a mammalian body, i.e. the compound localizes to a greater extent at the target site than to surrounding tissue.

As used herein, the term "peptide" refers to a molecule comprising an amino acid sequence of at least two amino acids.

As used herein, the term "amino acid sequence" is defined herein as a polyamide obtainable by polycondensation of at least two amino acids.

As used herein, the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" within the above definition.

An amide bond as used herein means any covalent bond having the structure 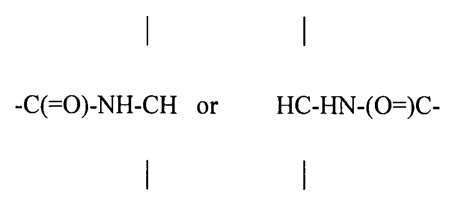 wherein the carbonyl group is provided by one molecule and the NH-group is provided by the other molecule to be joined. The amide bonds between two adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification, an amino acid residue is derived from the corresponding amino acid by forming a peptide bond with another amino acid.

For the purpose of the specification, an amino acid sequence may comprise naturally occurring and/or synthetic amino acid residues, proteinogenic and/or non-proteinogenic amino acid residues. The non-proteinogenic amino acid residues may be further classified as (a) homoanalogues of proteinogenic amino acids, (b) β-homoanalogues of proteinogenic amino acid residues and (c) further non-proteinogenic amino acid residues.

Accordingly, the amino acid residues are derived from the corresponding amino acids, e.g. from proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or non-proteinogenic amino acids, such as homoanalogues of proteinogenic amino acids wherein the sidechain has been extended by a methylene group, e.g. homoalanine (Hal), homoarginine (Har), homocysteine (Hcy), homoglutamine (Hgl), homohistidine (Hhi), homoisoleucine (Hil), homoleucine (Hle), homolysine (Hly), homomethionine (Hme), homophenylalanine (Hph), homoproline (Hpr), homoserine (Hse), homothreonine (Hth), homotryptophane (Htr), homotyrosine (Hty) and homovaline (Hva); β-homoanalogues of proteinogenic amino acids wherein a methylene group has been inserted between the α-carbon and the carboxyl group yielding β-amino acids, e.g. β-homoalanine (βHal), β-homoarginine (βHar), β-homoasparagine (βHas), β-homocysteine (βHcy), β-homoglutamine (βHgl), β-homohistidine (βHhi), β-homoisoleucine (βHil), β-homoleucine (βHle), β-homolysine (βHly), β-homomethionine (βHme), β-homophenylalanine (βHph), β-homoproline (βHpr), β-homoserine (βHse), β-homothreonine (βHth), β-homotryptophane (βHtr), β-homotyrosine (βHty) and β-homovaline (βHva);
further non-proteinogenic amino acids, e.g. α-aminoadipic acid (Aad), β-aminoadipic acid (βAad), α-aminobutyric acid (Abu), α-aminoisobutyric acid (Aib), β-alanine (βAla), 4-aminobutyric acid (4-Abu), 5-aminovaleric acid (5-Ava), 6-aminohexanoic acid (6-Ahx), 8-aminooctanoic acid (8-Aoc), 9-aminononanoic acid (9-Anc), 10-aminodecanoic acid (10-Adc), 12-aminododecanoic acid (12-Ado), α-aminosuberic acid (Asu), azetidine-2-carboxylic acid (Aze), β-cyclohexylalanine (Cha), citrulline (Cit), dehydroalanine (Dha), γ-carboxyglutamic acid (Gla), α-cyclohexylglycine (Chg), propargylglycine (Pra), pyroglutamic acid (Glp), α-tert-butylglycine (Tle), 4-benzoylphenylalanine (Bpa), δ-hydroxylysine (Hyl), 4-hydroxyproline (Hyp), allo-isoleucine (aIle), lanthionine (Lan), (1-naphthyl)alanine (1-Nal), (2-naphthyl)alanine (2-Nal), norleucine (Nle), norvaline (Nva), ornithine (Orn), phenylglycin (Phg), pipecolic acid (Pip), sarcosine (Sar), selenocysteine (Sec), statine (Sta), β-thienylalanine (Thi), 1,2,3,4-tetrahydroisochinoline-3-carboxylic acid (Tic), allo-threonine (aThr), thiazolidine-4-carboxylic acid (Thz), γ-aminobutyric acid (GABA), iso-cysteine (iso-Cys), diaminopropionic acid (Dpr), 2,4-diaminobutyric acid (Dab), 3,4-diaminobutyric acid (γ,βDab), biphenylalanine (Bip), phenylalanine substituted in para-position with -C₁-C₆-alkyl, -halide, -NH₂ or -CO₂H (Phe(4-R) wherein R = -C₁-C₆-alkyl, -halide, -NH₂, or -CO₂H); peptide nucleic acids (PNA, cf. P.E. Nielsen, Acc.Chem.Res. 32, 624-30)
or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification, a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

Examples for peptides which can be used as targeting agents in the compounds of the present invention are, but are not limited to, somatostatin and derivatives thereof and related peptides, neuropeptide Y and derivatives thereof and related peptides, bombesin and derivatives thereof and related peptides, gastrin, gastrin releasing peptide derivatives thereof and related peptides, epidermal growth factor (EGF of various origin), insulin growth factor (IGF) and IGF-1, LHRH agonists and antagonists, transforming growth factors, particularly TGF-α, angiotensin, cholecystokinin (CCK) and analogs, neurotensin and analogs, thyrotropin releasing hormone, prolactin, tumor necrosis factor, IL-1, IL-2, IL-4 or IL-6, interferons, VIP and related peptides, PACAP and related peptides. Such peptides comprise from 4 to 100 amino acids, wherein the amino acids are selected from natural and non-natural amino acids and also comprise modified natural and non-natural amino acids.

In other preferred embodiments, the targeting agent is selected to be an oligonucleotide.

For the purposes of this invention, the term "oligonucleotide" shall have the following meaning: short sequences of nucleotides, typically with twenty or fewer bases. Examples are, but are not limited to, molecules named and cited in: "The aptamers handbook. Functional oligonuclides and their application" by Svenn Klussmann, Wiley-VCH, 2006. An example for such an oligonucleotide is TTA1 (J. Nucl. Med., 2006, April, 47(4), 668-78).

For the purpose of this invention, the term "aptamer" refers to an oligonucleotide, comprising from 4 to 100 nucleotides, wherein at least two single nucleotides are connected to each other via
a phosphodiester linkage. Said aptamers have the ability to bind specifically to a target molecule (see e.g. M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). There are many ways known to the skilled person of how to generate such aptamers that have specificity for a certain target molecule. An example is given in WO 01/09390, the disclosure of which is hereby incorporated by reference. Said aptamers may comprise substituted or non-substituted natural and non-natural nucleotides. Aptamers can be synthesized in vitro using e.g. an automated synthesizer. Aptamers according to the present invention can be stabilized against nuclease degradation e.g. by the substitution of the 2'-OH group versus a 2'-fluoro substituent of the ribose backbone of pyrimidine and versus 2'-O-methyl substituents in the purine nucleic acids. In addition, the 3' end of an aptamer can be protected against exonuclease degradation by inverting the 3' nucleotide to form a new 5'-OH group, with a 3' to 3' linkage to a penultimate base.

For the purpose of this invention, the term "nucleotide" refers to molecules comprising a nitrogen-containing base, a 5-carbon sugar, and one or more phosphate groups. Examples of said base comprise, but are not limited to, adenine, guanine, cytosine, uracil, and thymine. Also non-natural, substituted or non-substituted bases are included. Examples of 5-carbon sugar comprise, but are not limited to, D-ribose, and D-2-desoxyribose. Also other natural and non-natural, substituted or non-substituted 5-carbon sugars are included. Nucleotides as used in this invention may comprise from one to three phosphates.

Small molecules effective for targeting certain sites in a biological system can also be used as the targeting agent.

For the purposes of this invention, the term "small molecule" shall have the following meaning: a small molecule is a compound that has a molecular mass of 200 to 800 and that contains a primary and/or secondary amine, a thiol or hydroxyl group coupled via **L** to rest of the molecule in the compounds of Formulae I, II and III. Such targeting moieties are known in the art, so are methods for preparing them.

Specific examples for targeting agents may comprise, without being limited thereto, sugars, oligosaccharides, polysaccharides, amino acids, nucleic acids, nucleotides, nucleosides, oligonucleotides, proteins, peptides, peptidomimetics, antibodies, aptamers, lipids, hormones (steroid and nonsteroid), neurotransmitters, drugs (synthetic or natural), receptor agonists and antagonists, dendrimers, fullerenes, virus particles and other targeting molecules (e.g. cancer targeting molecules).

As used herein the term "pharmaceutically acceptable salt" comprises salts of inorganic and organic acids such an mineral acids, including, but not limited to, acids such as carbonic, nitric or sulfuric acid, or organic acids, including, but not limited to acids such as aliphatic, cycloaliphatic, aromatic, araliphatic, heterocyclic, carboxylic and sulphonic acids, examples of which are formic, acetic, trifluoroacetic, propionic, succinic, glycolic, gluconic, lactic, malic, fumaric, pyruvic, benzoic, anthranilic, mesylic, salicylic, phenylacetic, mandelic, embonic, methansulfonic, ethanesulfonic, benzenesulfonic, phantothenic, toluenesulfonic and sulfanilic acid.

If a chiral center or another form of an isomeric center is present in a compound according to Formula I, II or III of the present invention, all forms of such isomers, including enantiomers and diastereoisomers, are intended to be covered herein. Compounds containing a chiral center may be used as a racemic mixture or as an enantiomerically enriched mixture, or the racemic mixture may be separated using well-known techniques and an individual enantiomer maybe used alone. In cases in which compounds have unsaturated carbon-carbon double bonds, both the cis-isomer and trans-isomers are within the scope of this invention. In cases in which compounds may exist in tautomeric forms, such as keto-enol tautomers, each tautomeric form is contemplated as being included within this invention whether existing in equilibrium or predominantly in one form.

Abbreviations used throughout the specification are used within the following meanings:
- Ts: tosyl
- Ns: nitrophenylsulfenyl
- Cbz: carbobenzoxy
- Bz: benzoyl
- Bn: benzyl
- Boc: tert-butoxycarbonyl
- Fmoc: 9-fluorenylmethoxycarbonyl
- Tr: triphenylmethyl

### Detailed Description of Invention

Examples of the preparation/synthesis of precursor compounds are shown below and are illustrative of some of the embodiments of the invention described herein. These examples should not be considered to limit the spirit or scope of the invention in any way. The aziridine moiety of these precursors can easily be halogenated, such as fluorinated with ¹⁸F.

### Methods of preparing compounds according to formulae I, II and III

Scheme 1 shows a possible way for synthesis of compounds of Formula I.

Compounds of formula I can be synthesised starting with commercial aziridines **1** or from α-amino alcohols via mesylation or tosylation of the alcohol and nucleophilic substitution towards the formation of aziridines **1** (not shown). Depending on the substitution pattern on the aziridine, it might be necessary to perform the first steps of appropriate functionalisation with an inert protecting group as trityl. If the substitution pattern leads to a more stable aziridine, electron deficient activation groups as needed for halogenation or fluorination, respectively might be included straight from the beginning of the synthetic sequence. In the procedure shown here, the aziridine is protected first with a trityl group followed by saponification of the methyl ester **2.** The resulting acid **3** can be converted to an active ester **4** followed by treatment with glycine or directly coupled with glycine to yield the aziridine derivative **5** with an extended linker. This is necessary if n=0 as aziridines directly substituted with a carboxylate functionality are less stable then aziridines substituted with amides. This linker extension is not necessary if n>0. In the next step the trityl protection is cleaved and several other groups **(6),** preferably substituted aryl sulfonyl groups can be introduced to activate the aziridine towards nucleophilic substitution (fluorination). Saponification to **7** leads to building blocks which can be added to targeting agents to give labelling precursors **8**.

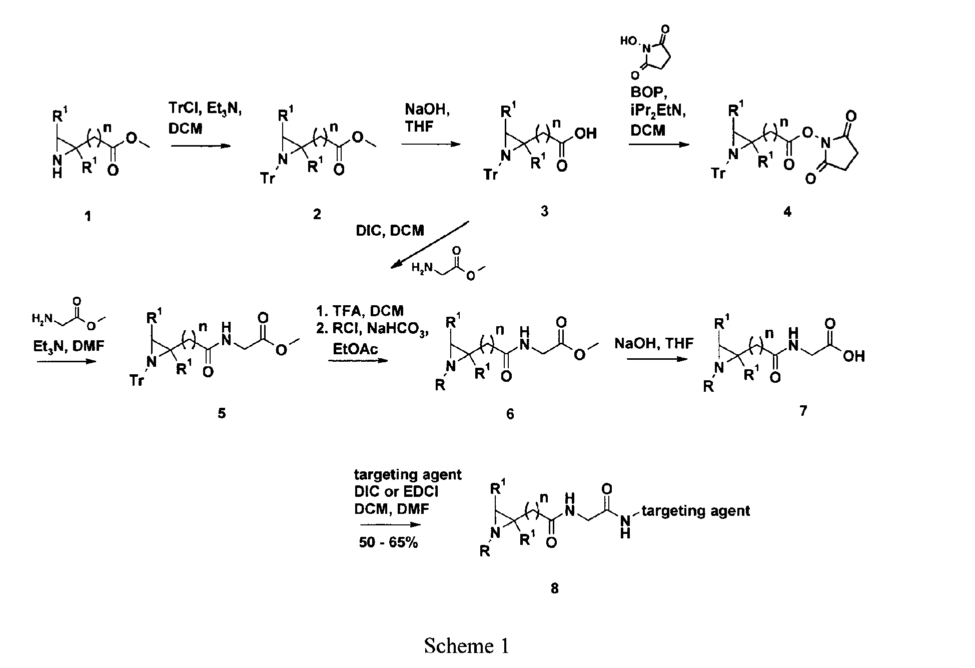

Scheme 2 shows a possible way of synthesis of compounds according to Formula II

Compounds of formula II can be synthesised starting with appropriate substituted aryl derivatives **13** by introducing a chlorosulfonyl group towards **14** followed by the addition of commercially available neat aziridine to give the substituted aziridine **15.** Saponification leads to building blocks **16** which can be added to targeting agents to give labelling precursors **17.**

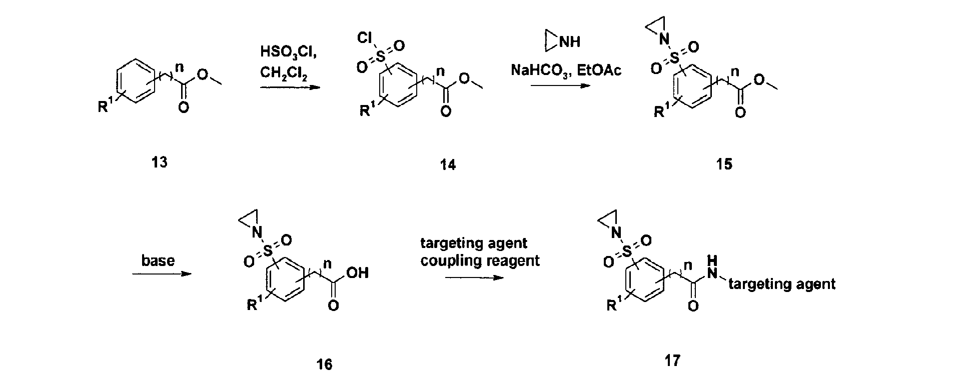

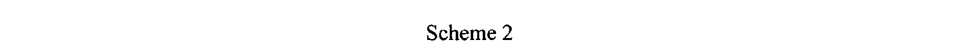

Scheme 3 shows a possible way of synthesis of compounds according to formula III.

Compounds of formula III can be synthesised starting with the reaction of a dihydro pyrrole **20** and methyl 4-chloro-4-oxybutyrate **21** which leads to the substituted dihydro pyrrole **22.** The following steps as epoxidation **(23),** opening of the epoxide with azide **(24),** tosylation of the resulting alcohol **(25),** Staudinger reduction of the azide followed by substitution of the tosylate **(26)** are used to generate the desired aziridine **26.** Different types of activating groups R, preferably substituted aryl sulfonyl groups can be introduced to give **27.** Saponification leads to building blocks **28** which can be added to targeting agents directly or via an active ester **29** to give labelling precursors **30.**

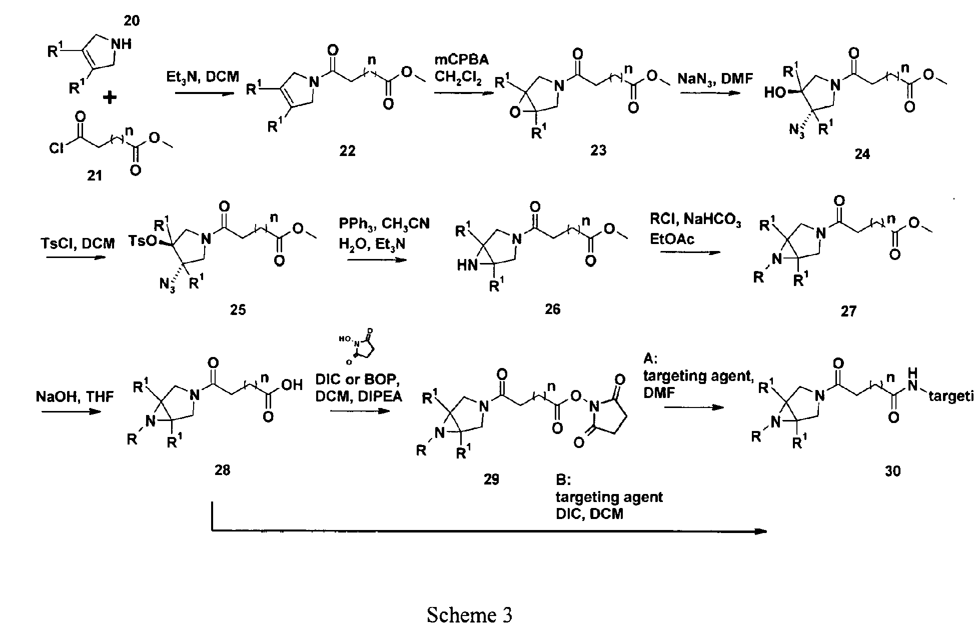

Experimental details can be seen from the experimental part hereinafter.

The fluorination reaction leading to labelled derivatives, as typical examples of halogenation reactions of all such different types of aziridine compounds is shown in Scheme 4.

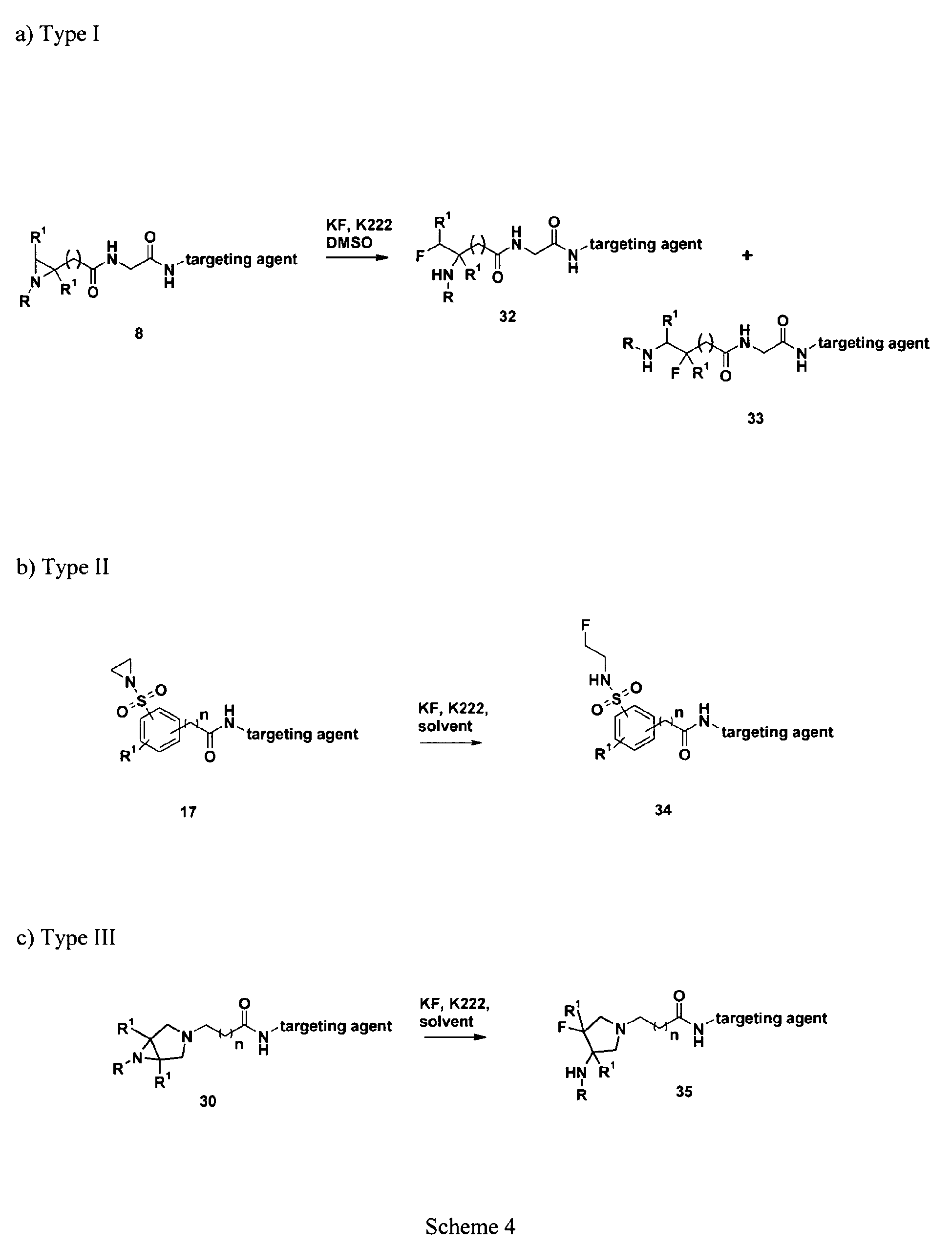

### EXPERIMENTAL PART

### Example 1

### Preparation of compounds according formula I and corresponding model compounds

### Preparation according to scheme 1 with n = 0.

### Preparation of 1-Trityl-aziridine-2-carboxylic acid methyl ester 2a

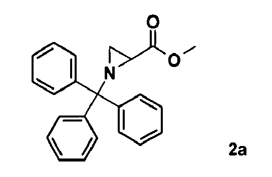

3 g (29.6 mmol) aziridine **1a** was solved in 50 ml dichloromethane, cooled down to 0°C followed by the addition of 6.17 ml (44,51 mmol) triethylamine and 9.93 g (35.61 mmol) trityl chloride. The reaction mixture was stirred at rt for 2h and concentrated. The residue was purified by chromatography on silica gel to give 9.96 g (98%) of **2a**.
¹H-NMR (CDCl₃): δ = 7.41 (m, 6H), 7.30-7.17 (m, 9H), 3.77 (s, 3H), 2.26 (dd, 1H), 1.89 (dd, 1H), 1.42 (dd, 1H) ppm.

### Preparation of 1-Trityl-aziridine-2-carboxylic acid 3a

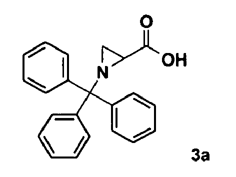

7,45 g (21.69 mmol) **2a** were solved in 55 ml tetrahydrofurane, cooled down to °C and treated with 34,7 ml (34.71 mmol) 1 N sodium hydroxide solution. The reaction mixture was stirred overnight a rt, concentrated and the residue was purified by chromatography on silica gel to give 6.91 g (97%) of **3a**.
¹H-NMR (MeOD): δ = 7.45 (m, 6H), 7.30-7.17 (m, 9H), 2.16 (dd, 1H), 1.78 (dd, 1H), 1.40 (dd, 1H) ppm.

### Preparation of 1-Trityl-aziridine-2-carboxylic acid-2,5-dioxo-pyrrolidin-1-yl ester 4a

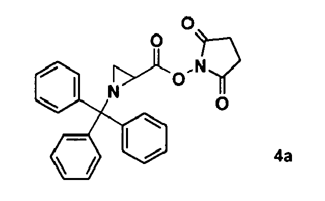

910 mg (2.76 mmol) **3a** were solved in dichloromethane, 1.34 g (3.04 mmol) BOP and 318 mg (2.76 mmol) N-hydroxysuccinimide were added and the solution was cooled down to 0°C. Then 0,76 ml (4.42 mmol) ethyl diisopropylamine was added slowly and the reaction was stirred overnight at rt. The reaction mixture was diluted with dichloromethane, washed with 10% citric acid and brine, dried over sodium sulfate and concentrated. The residue was purified by chromatography on silica gel to give 760 mg (64%) of **4a**.
¹H-NMR (MeOD): δ = 7.45 (m, 6H), 7.30-7.17 (m, 9H), 2.84 (s, 4H), 2.44 (m, 1H), 2.09 (dd, 1H), 1.60 (dd, 1H) ppm.

### Preparation of {[1-(Trityl)-aziridine-2-carbonyl]-amino} acetic acid methyl ester 5a

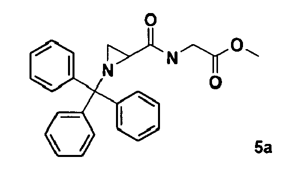

218 mg (1.74 mmol) glycin methylester hydrochloride was solved in DMF and treated with 0,36 ml (2.6 mmol) triethyl amine. After 30 min at rt 740 mg (1.74 mmol) **4a** was added. The reaction mixture was stirred for 2h at 50°C and then concentrated. The residue was purified by chromatography on silica gel to give 550 (79%) of **5a.**
¹H-NMR (CDCl₃): δ = 7.45 (m, 6H), 7.30-7.17 (m, 9H), 4.22 (dd, 1H), 4.10 (dd, 1H), 3.81 (s, 3H), 2.05 (m, 2H), 1.50 (dd, 1H) ppm.

### Preparation of {[1-(Toluene-4-sulfonyl)-aziridine-2-carbonyl]-amino} acetic acid methyl ester 6aa

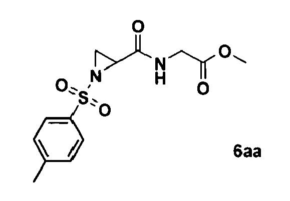

2.3 g (5.74 mmol) **5a** was solved in 95 ml chloroform, cooled down to 0°C and titrated with trifluoro acetic acid until complete conversion. The mixture was neutralized with saturated sodium bicarbonate solution and concentrated. The residue was suspended in 95 ml ethyl acetate and 95 ml saturated sodium bicarbonate solution followed by (11.49 mmol) sulfonic acid chloride. The reaction mixture was stirred overnight at rt. The phases were separated, the aqueous phase was extracted with ethyl acetate and the combined organic phases were dried over sodium sulphate and concentrated. The residue was purified by chromatography on silica gel to give (21-47%) of **6aa.**
¹H-NMR (MeOD): δ = 7.83 (d, 2H), 7.45 (d, 2H), 3.89 (s, 2H), 3.67 (s, 3H), 3.30 (d, 1H), 2.76 (d, 1H), 2.50 (d, 1H), 2.44 (s, 3H) ppm.

### Preparation of {[1-(2,4,6-Triisopropyl-benzenesulfonyl)-aziridine-2-carbonyl]-amino} acetic acid methyl ester 6ab

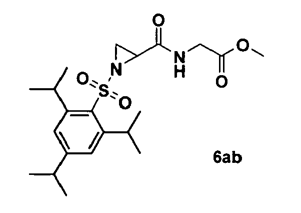

This compound was prepared in an analogous way to **6aa**.
¹H-NMR (MeOD): δ = 7.33 (s, 2H), 4.33 (sept, 2H), 3.98 (d, 2H), 3.73 (s, 3H), 3.43 (dd, 1H), 2.98 (sept, 1H), 2.87 (d, 1H), 2.60 (d, 1H), 1.32-1.28 (m, 18H) ppm.

### Preparation of {[1-(3,4-Dimethoxy-benzenesulfonyl)-aziridine-2-carbonyl]-amino} acetic acid methyl ester 6ac

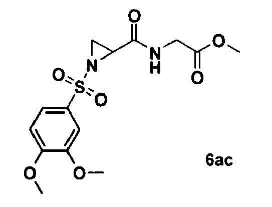

This compound was prepared in an analogous way to **6aa**.
¹H-NMR (CDCl₃): δ = 7.56 (dd, 1H), 7.41 (d, 1H), 7.00 (d, 1H), 6.62 (bt, 1H), 4.03 (dd, 1H), 3.97 (s, 3H), 3.92 (dd, 1H), 3.73 (s, 3H), 3.28 (dd, 1H), 2.83 (d, 1H), 2.46 (d, 1H) ppm.

### Preparation of {[1-(Toluene-4-sulfonyl)-aziridine-2-carbonyl]-amino} acetic acid 7aa

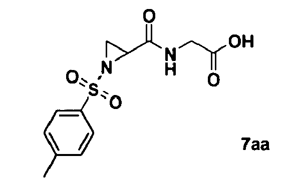

(1.18 mmol) **6aa** was solved in 15 ml tetrahydrofurane, cooled down to 0°C and treated with 0.71 ml (1.42 mmol) 2N sodium hydroxide solution. The reaction mixture was stirred at rt for 2h and concentrated. The residue was taken up in water, carefully neutralized with citric acid and extracted with ethyl acetate. The combined organic phases were washed with brine, dried over sodium sulphate, filtrated and concentrated. The product **7aa** (90-97%) was used without further purification.
¹H-NMR (MeOD): δ = 7.83 (d, 2H), 7.44 (d, 2H), 3.86 (s, 2H), 3.30 (d, 1H), 2.76 (d, 1H), 2.51 (d, 1H), 2.44 (s, 3H) ppm.

### Preparation of {[1-(2,4,6-Triisopropyl-benzenesulfonyl)-aziridine-2-carbonyl]-amino} acetic acid 7ab

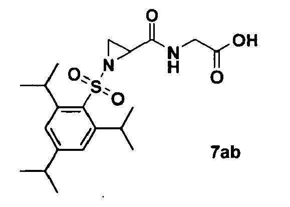

This compound was prepared in an analogous way to **7aa**.
¹H-NMR (MeOD): δ = 7.27 (s, 2H), 4.27 (sept, 2H), 3.90 (d, 2H), 3.99 (dd, 1H), 2.93 (sept, 1H), 2.78 (d, 1H), 2.55 (d, 1H), 1.30-1.23 (m, 18H) ppm.

### Preparation of {[1-(3,4-Dimethoxy-benzenesulfonyl)-aziridine-2-carbonyl]-amino} acetic acid 7ac

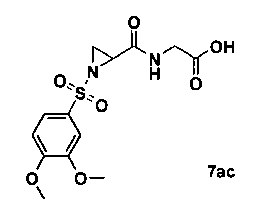

This compound was prepared in an analogous way to **7aa**.
¹H-NMR (CDCl₃): δ = 7.56 (dd, 1H), 7.39 (d, 1H), 6.99 (d, 1H), 6.78 (bt, 1H), 4.09 (dd, 1H), 3.96 (s, 3H), 3.94 (dd, 1H), 3.95 (s, 3H), 3.32 (dd, 1H), 2.80 (d, 1H), 2.46 (d, 1H) ppm.

### Preparation of 1-(Toluene-4-sulfonyl)-aziridine-2-carboxylic acid - Gly-Val-βAla-Phe-Gly-amide 8aaa

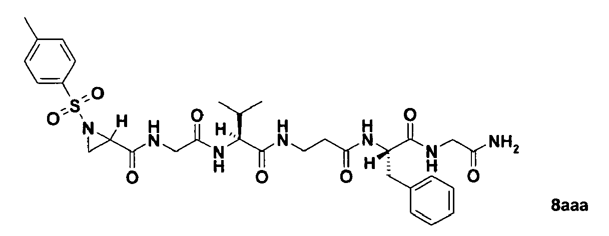

0.1 mmol resin bound di- or tetrapeptide, swolen in DMF was filtered and added to a solution of 0.3 mmol **7aa,** 113.7 mg (0.3 mmol) HBTU and 104.5 µl (0.6 mmol) diisopropyl ethyl amine in 1.5 ml DMF. The mixture was shaken for 4h, filtered and the remaining resin was washed with DMF and dichloromethane and dried under vacuum. Then the resin was treated with 1.5 ml of a mixture containing 85% TFA, 5% water, 5% phenol and 5% triisopropyl silane for 2h, filtered followed by precipitation of the product in 20 ml MTBE. The precipitate was purified by HPLC to give 7-23% **8aaa**.
HPLC-MS (ES+): *m*/*z* (%) = 672 (100).

### Preparation of 1-(2,4,6-Triisopropyl-benzenesulfonyl)-aziridine-2-carboxylic - Gly-Val-βAla-Phe-Gly-amide 8aba

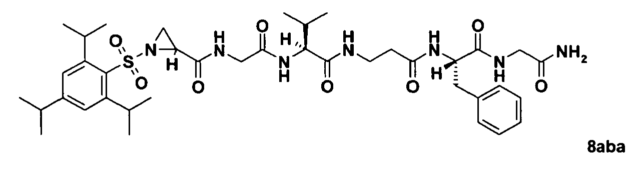

This compound was prepared in an analogous way to **8aaa** starting from **7ab**.

HPLC-MS (ES+): *m*/*z* (%) = 784 (100).

### Preparation of 1-(2,4,6-Triisopropyl-benzenesulfonyl)-aziridine-2-carboxylic acid [(3-{3-[(2R,4S,5R)-4-(1-methoxy-cyclohexyloxy)-5-(1-methoxy-cyclohexyloxymethyl)-tetrahydrofuran-2-yl]-5-methyl-2,6-dioxo-3,6-dihydro-2H-pyrimidin-1-yl}-propylcarbamoyl)-methyl]-amide 8abb

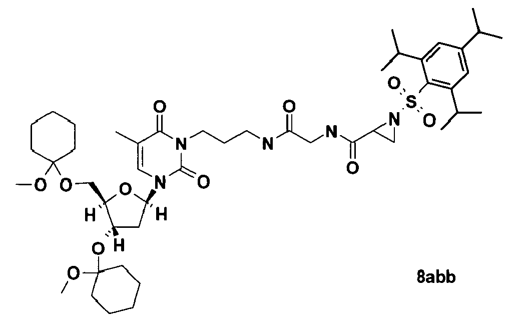

60 mg (0.15 mmol) **7ab** were solved in 4 mL dichloromethane followed by 46 µL (0.29 mmol) DIC and 76.5 mg (0.15 mmol) 3-(3-Amino-propyl)-1-[(2R,4S,5R)-4-(1-methoxy-cyclohexyloxy)-5-(1-methoxy-cyclohexyloxymethyl)-tetrahydro-furan-2-yl]-5-methyl-1H-pyrimidine-2,4-dione. The reaction mixture was stirred over night at rt and concentrated. The residue was purified by chromatography on silica gel to give 87 mg (65%) of **8abb**.
¹H-NMR (CDCl₃): δ = 7.56 (s, 1H), 7.21 (s, 2H), 7.01 (t, 1H), 6.72 (t, 1H), 6.35 (t, 1H), 4.51 (m, 1H), 4.28 (sept, 2H), 4.16 (m, 1H), 4.08 (dd, 1H), 3.97 (m, 2H), 3.76 (dd, 1H), 3.67 (dd, 1H), 3.57 (dd, 1H), 3.44 (dd, 1H), 3.21 (s, 3H), 3.18 (s, 3H), 3.16 (m, 2H), 2.92 (sept, 1H), 2.86 (d, 1H), 2.66 (d, 1H), 2.36 (m, 1H), 2.05 (dd, 1H), 1.91 (s, 3H), 1.82-1.76 (m, 6H), 1.54-1.37 (m, 14H), 1.30-1.26 (div. d, 18H) ppm.

### Preparation of model compounds to test fluorination

### Preparation of 1-Trityl-aziridine-2-carboxylic acid benzylamide 9a

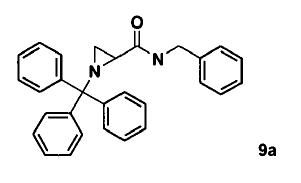

6 g (14.07 mmol) **4a** was solved in 300 ml dichloromethane, followed by the addition of 1.57 ml (14.07 mmol) benzylamine. The reaction mixture was stirred overnight at rt and concentrated. The residue was purified by chromatography on silica gel to give 3.27 (55%) of **9a**.
¹H-NMR (CDCl₃): δ = 7.43-7.20 (m, 20H), 7.12 (t, 1H), 4.76 (dd, 1H), 4.35 (dd, 1H), 2.09 (dd, 1H), 2.02 (d, 1H), 1.52 (d, 1H) ppm.

### Preparation of 1-(Toluene-4-sulfonyl)aziridine-2-carboxylic acid benzylamide 10aa

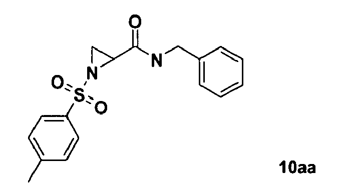

220 mg (0.53 mmol) **9a** was solved in chloroform, cooled down to 0°C and titrated with trifluoro acetic acid until complete conversion. Saturated sodium bicarbonate solution was added until pH 6-7 was reached and the solution was concentrated. The residue was taken up in 15 ml ethyl acetate, treated with 15 ml saturated sodium bicarbonate solution followed by (1.05 mmol) sulfonic acid chloride. The reaction mixture was stirred overnight at rt. The organic phase was separated, dried over sodium sulphate and concentrated. The residue was purified by chromatography on silica gel to give (43-65%) of **10aa.**
¹H-NMR (CDCl₃): δ = 7.81 (d, 2H), 7.36 (d, 2H), 7.29-7.26 (m, 4H), 7.10 (dd, 2H), 6.41 (bt, 1H), 4.36 (dd, 1H), 3.30 (dd, 1H), 2.93 (d, 1H), 2.47 (s, 3H), 2.41 (d, 1H) ppm.

### Preparation of 1-(2,4,6-Triisopropyl-benzenesulfonyl)-aziridine-2-carboxylic acid benzyl amide 10ab

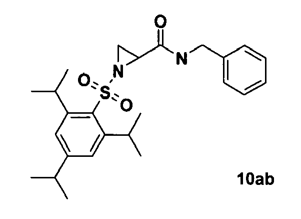

This compound was prepared in an analogous way to **10aa.**
¹H-NMR (CDCl₃): δ = 7.35-7.27 (m, 4H), 7.17 (s, 2H), 7.15 (m, 1H), 6.32 (t, 1H), 4.37 (dd, 1H), 4.35 (dd, 1H), 4.20 (sept, 2H), 3.42 (dd, 1H), 2.91 (sept, 1H), 2.87 (d, 1H), 2.38 (d, 1H), 1.26 (d, 6H), 1.19 (2d, 12H) ppm.

### Preparation of 1-(3,4-dimethoxy-benzenesulfonyl)-aziridine-2-carboxylic acid benzyl amide 10ac

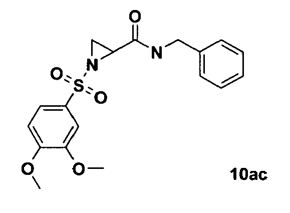

This compound was prepared in an analogous way to **10aa.**
¹H-NMR (CDCl₃): δ = 7.51 (dd, 1H), 7.34-7.26 (m, 5H), 7.11-7.08 (m, 1H), 6.97 (d, 1H), 6.41 (bt, 1H), 4.39 (dd, 1H), 4.33 (dd, 1H), 3.97 (s, 3H), 3.89 (s, 3H), 3.29 (dd, 1H), 2.83 (d, 1H), 2.42(d,1H)ppm.

### Fluorination of model compounds

### Preparation of N-Benzyl-3-fluoro-2-(toluene-4-sulfonylamino)-propionamide 11aa

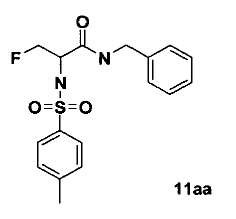

0.079 mmol of **10aa** was solved in DMSO followed by the addition of 32.75 mg (0.087 mmol) Kryptofix 222 and 5.05 mg (0.87 mmol) KF. The reaction mixture was stirred at 50°-80°C for 1h, taken up in ethyl acetate and extracted with saturated ammonium chloride solution. The combined aqueous phases were extracted twice with ethyl acetate. The combined organic phases were washed with brine, dried over sodium sulphate, filtered and concentrated. The residue was purified by chromatography on silica gel to give (23-71%) of **11aa.**
¹H-NMR (CDCl₃): δ = 7.76 (d, 2H), 7.38-7.26 (m, 5H), 7.19 (d, 2H), 6.72 (bt, 1H), 5.41 (d, 1H), 4.84 (ddd, 1H), 4.45 (dd, 1H), 4.40 (dd, 1H), 4.20 (ddd, 1H), 3.95 (m, 1H), 2.44 (s, 3H) ppm.

### Preparation of N-Benzyl-3-fluoro-2-(2,4,6-triisopropyl-benzenesulfonylamino)-propionamide 11ab

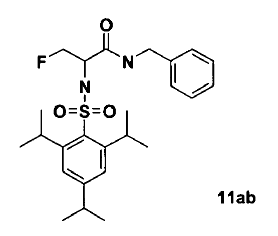

This compound was prepared in an analogous way to **11aa.**
¹H-NMR (CDCl₃): δ = 7.35-7.16 (m, 7H), 6.84 (bt, 1H), 5.40 (d, 1H), 4.90 (ddd, 1H), 4.51 (dd, 1H), 4.40 (dd, 1H), 4.25 (ddd, 1H), 4.06 (m, 1H), 4.02 (sept, 2H), 2.91 (sept, 1H), 1.19 (m, 18H) ppm.

### Preparation of N-Benzyl-2-(3,4-dimethoxy-benzenesulfonylamino)-3-fluoro-propionamide 11ac

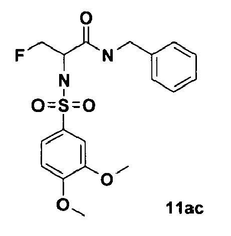

This compound was prepared in an analogous way to **11aa.**
¹H-NMR (CDCl₃): δ = 7.48 (dd, 1H), 7.34-7.26 (m, 5H), 7.18 (d, 1H), 6.92 (d, 1H), 6.71 (bt, 1H), 5.43 (d, 1H), 4.84 (ddd, 1H), 4.45 (dd, 1H), 4.41 (dd, 1H), 4.26 (ddd, 1H), 3.95 (s, 3H), 3.93 (m, 1H), 3.90 (s, 3H) ppm.

### Fluorination

### Preparation of 3-Fluoro-2-(2,4,6-triisopropyl-benzenesulfonylamino)-propion- Gly-Val-βAla-Phe-Gly-amide 32aba

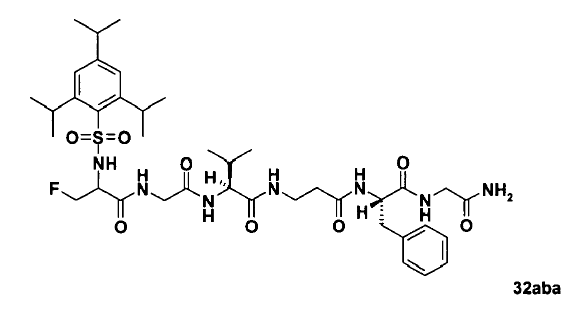

4 mg (5.1 µM) aziridine **8aba** were treated with a mixture of 1.2 mg (20.4 µM) KF and 7.7 mg (20.4 µM) Kryptofix in 0.5 mL DMSO for 15 min at 50°C. Then the reaction mixture was analyzed by HPLC-MS which showed a conversion of 10% to the desired product **32aba.**
HPLC-MS (ES+): *m*/*z* (%) = 804.14 (100).

### Preparation of 3-Fluoro-N-[(3-{3-[(2R,4S,SR)-4-(1-methoxy-cyclohexyloxy)-5-(1-methoxy-cyclohexyloxymethyl)-tetrahydro-furan-2-yl]-5-methyl-2,6-dioxo-3,6-dihydro-2H-pyrimidin-1-yl}-propylcarbamoyl)-methyl]-2-(toluene-4-sulfonylamino)-propionamide 32abb

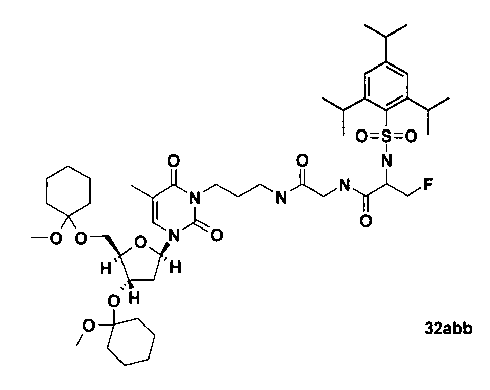

30 mg (0.033 mmol) **8abb** were solved in 1.5 mL DMSO followed by 13.6 mg (0.036 mmol) Kryptofix K222 and 2.1 mg (0.036 mml) KF. The reaction mixture was stirred at 50°C for 30 min until complete conversion of the starting material. The mixture was then diluted with ethyl acetate, washed with saturated aqueous ammonium chloride solution, brine, dried over sodium sulphate, filtrated and concentrated. The residue was purified by chromatography on silica gel to give 5 mg (16%) of **32abb.**
¹H-NMR (CDCl₃): δ = 7.62 (s, 1H), 7.52 (dd, 1H), 7.30 (t, 1H), 7.18 (s, 2H), 6.84 (d, 1H), 6.34 (dd, 1H), 4.89 (ddd, 1H), 4.51 (m, 1H), 4.42 (ddd, 1H), 4.32 (dd, 1H), 4.19-4.15 (m, 2H), 4.11 (sept, 2H), 4.01-3.97 (m, 2H), 3.71-3.66 (m, 2H), 3.57 (dd, 1H), 3.30 (m, 1H), 3.21 (s, 3H), 3.18 (s, 3H), 3.02 (m, 1H), 2.91 (sept, 1H), 2.40 (ddd, 1H), 2.07-2.03 (m, 2H), 1.88 (s, 3H), 1.86-1.83 (m, 2H), 1.80-1.72 (m, 4H), 1.60-1.45 (m, 16H), 1.27-1.24 (div. d, 18H) ppm.

### Example 2

### Preparation of compounds according formula II and corresponding model compounds

### Preparation according to Scheme 2 with n = 1.

### Preparation of (2-Chlorosulfonyl-3,5-dimethoxy-phenyl)-acetic acid methyl ester 14a

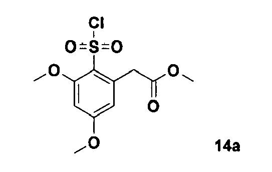

0.4 mL (6 mmol) Chlorosulfonic acid were solved in 4 mL dichloromethane at -10°C followed by the slow addition of 600 mg (2.85 mmol) (3,5-Dimethoxy-2-methyl-phenyl)-acetic acid methyl ester **13a** solved in 2 mL dichloromethane. The reaction mixture was stirred for 1h at rt, diluted with 50 mL acetic acid ethyl ester and washed with 10 mL saturated sodium bicarbonate solution. The phases were separated and the aqueous phase was extracted with acetic acid ethyl ester. The combined organic phases were washed with brine, dried over sodium sulphate, filtrated and concentrated to give 451 mg (51%) of crude **14a** which was used in the next step without further purification.
¹H-NMR (CDCl₃): δ = 6.54 (d, 1H), 6.37 (d, 1H), 4.03 (s, 5H), 3.89 (s, 3H), 3.72 (s, 3H).

### Preparation of [2-(Aziridine-1-sulfonyl)-3,5-dimethoxy-phenyl]-acetic acid methyl ester 15a

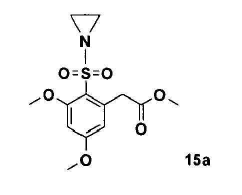

0.22 mL (4.2 mmol) aziridine were solved at 0°C in a mixture of 3.5 mL saturated sodium bicarbonate solution and 7 mL ethyl acetate followed by the addition of 432 mg (1.4 mmol) (2-Chlorosulfonyl-3,5-dimethoxy-phenyl)-acetic acid methyl ester **14a.** The reaction mixture was then stirred at rt for 1h. The phases were separated and the aqueous phase was extracted with ethyl acetate. The combined organic phases were washed with brine, dried over sodium sulphate, filtrated and concentrated. The residue was purified by chromatography on silica gel to give 307 mg (70%) of **15a**.
¹H-NMR (CDCl₃): δ = 6.52 (d, 1H), 6.38 (d, 1H), 4.05 (s, 2H), 3.95 (s, 3H), 3.86 (s, 3H), 3.71 (s, 3H), 2.44 (s, 4H) ppm.

### Example 3

### Preparation of compounds according formula III and corresponding model compounds

### Preparation according to Scheme 3 with n = 1.

### Preparation of 4-(2,5-Dihydro-pyrrol-1-yl)-4-oxo-butyric acid methyl ester 22a

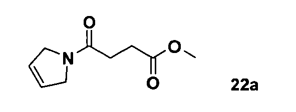

1 g (22.14 mmol) 2,5-dihydro pyrrole **20** was solved in 60 ml dichloromethane and cooled down to 0° followed by the slow addition of 3.3 ml (26.57 mmol) methyl 4-chloro-4-oxobutyrate 21a and 4.6 ml (33.21 mmol) triethylamine. The reaction mixture was stirred at rt for 2 h and concentrated. The residue was purified by chromatography on silica gel to give 2.42 g (60%) of 22a.
¹H-NMR (CDCl₃): δ = 5.86 (m, 1H), 5.80 (m, 1H), 4.28-4.21 (m, 4H), 3.69 (s, 3H), 2.70 (m, 2H), 2.57 (m, 2H) ppm.

### Preparation of 4-(6-Oxa-3-aza-bicyclo[3.1.0]hex-3-yl)-4-oxo-butyric acid methyl ester 23a

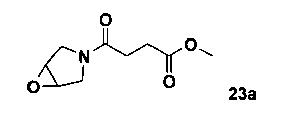

2.24 g (12.23mmol) **22a** was solved in 70 ml dichloromethane followed by the addition of 4.9 g (22.01 mmol, 77%) mCPBA. The reaction mixture was stirred at rt for 4d, diluted with ethyl acetate, washed with bicarbonate and brine, dried over sodium sulphate, filtered and concentrated. The residue was purified by chromatography on silica to give 1.34 g (55%) of 23a.
¹H-NMR (CDCl₃): δ = 4.01 (d, 1H), 3.86 (d, 1H), 3.82 (dd, 1H), 3.78 (dd, 1H), 3.73 (s, 3H), 3.62 (d, 1H), 3.44 (d, 1H), 2.82-2.52 (m, 4H) ppm.

### Preparation of 4-((3S,4S)-3-Azido-4-hydroxy-pyrrolidin-1-yl)-4-oxo-butyric acid methyl ester 24a

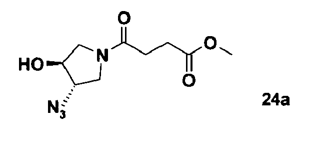

7.6 g (38.15 mmol) epoxide **23a** was solved in 250 ml DMF and treated with 3.47 g (53.41 mmol) sodium azide. The reaction mixture was stirred at 100°C for 5 h, cooled down, diluted with dichloromethane, washed with water and brine, dried over sodium sulphate, filtered and concentrated to give 4.6 g (50%) of crude **24a** which was used without further purification.
¹H-NMR (CDCl₃, mixture of diastereomers): δ = 4.26 (m, 1H), 4.03 (m, 1H), 3.88-3.44 (m, 4H), 3.67 (s, 3H), 2.70-2.51 (m, 4H) ppm.

### Preparation of 4-[(3S,4S)-3-Azido-4-(toluene-4-sulfonyloxy)-pyrrolidin-1-yl]-4-oxo-butyric acid methyl ester 25a

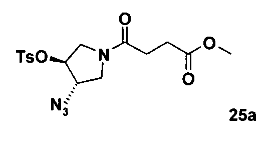

3.91 g (16.14 mmol) **24a** was solved in dichloromethane, cooled down to 0°C followed by the addition of 5.6 ml (40.35 mmol) triethylamine, 590 mg (4.84 mmol) DMAP and 5.39 g (28.25 mmol) tosyl chloride. The reaction mixture was stirred at rt overnight, concentrated, taken up in ethyl acetate, washed with saturated ammonium chloride solution and brine, dried over sodium sulphate, filtered and concentrated. The residue was purified by chromatography on silica gel to give 4.07 g (64%) of **25a**.
¹H-NMR (CDCl₃, mixture of diastereomers): δ = 7.82 (d, 2H), 7.79 (d, 2H), 7.41 (d, 2H), 7.38 (d, 2H), 4.83 (m, 1H), 4.76 (m, 1H), 4.33 (m, 1H), 4.13 (m, 1H), 3.83-3.79 (m, 2H), 3.68 (s, 6H), 3.70-3.53 (m, 6H), 2.66 (m, 4H), 2.56-2.46 (m, 4H), 2.48 (s, 3H), 2.47 (s, 3H) ppm.

### Preparation of 4-(3,6-Diaza-bicyclo[3.1.0]hex-3-yl)-4-oxo-butyric acid methyl ester 26a

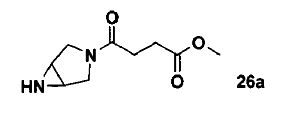

820 mg (2.07 mol) **25a** were solved in 32 ml acetonitrile followed by 564 mg (2.14 mmol) triphenyl phosphine. The reaction mixture was stirred at rt for 2.5 h followed by the addition of 0.9 ml (49 mmol) water. After stirring at rt overnight 0.8 ml (5.77 mmol) triethyl amine were added and the mixture was stirred another 5 h at rt and then concentrated. The residue was purified by chromatography on NH2-silica gel to give 263 mg (64%) of **26a**.
¹H-NMR (CDCl₃): δ = 3.91 (d, 1H), 3.74 (d, 1H), 3.68 (s, 3H), 3.55 (d, 1H), 3.42 (d, 1H), 2.80-2.72 (bm, 2H), 2.65 (dd, 2H), 2.51 (dd, 2H) ppm.

### Preparation of 4-Oxo-4-[6-(2,4,6-triisopropyl-benzenesulfonyl)-3,6-diaza-bicyclo[3.1.0]hex-3-yl]-butyric acid methyl ester 27a

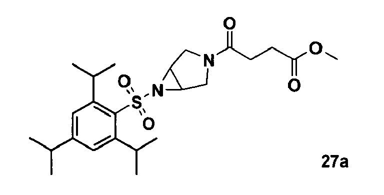

250 mg (1.26 mmol) **26a** was solved in 24 ml ethyl acetate and 24 ml saturated sodium bicarbonate solution followed by 764 mg (2.52 mmol) 2,4,6-triisopropyl phenyl sulfonyl chloride. The reaction mixture was stirred over night followed by phase separation and extraction of the aqueous phase with ethyl acetate. The combined organic phases were washed with brine, dried over sodium sulphate, filtrated and concentrated. The residue was purified by chromatography on silica to give 265 mg (45%) of **27a**.
¹H-NMR (CDCl₃): δ = 7.18 (s, 2H), 4.28 (sept, 2H), 3.94 (d, 1H), 3.79 (d, 1H), 3.70 (dd, 1H), 3.67 (s, 3H), 3.62 (dd, 1H), 3.58 (dd, 1H), 3.42 (dd, 1H), 2.91 (sept, 1H), 2.72-2.40 (m, 4H), 1.27-1.24 (m, 18H) ppm.

### Preparation of 4-Oxo-4-[6-(2,4,6-triisopropyl-benzenesulfonyl)-3,6-diaza-bicyclo[3.1.0]hex-3-yl]-butyric acid 28a

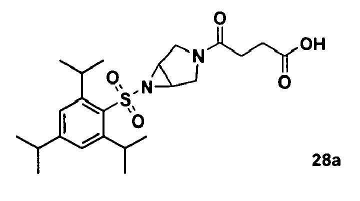

30 mg (0.065 mmol) **27a** were solved in 1 mL THF, cooled down to 0°C and treated with 0.045 mL 2N NaOH. The reaction mixture was stirred at room temperature for 5h, concentrated, diluted with water and the pH was adjusted at 4 with 10% aqueous citric acid. The aqueous solution was extracted several times with ethyl acetate. The combined organic phases were washed with brine, dried over sodium sulphate and concentrated to give 28 mg (96%) of **28a** which was used without further purification.
¹H-NMR (CDCl₃): δ = 7.18 (s, 2H), 4.27 (sept, 2H), 3.95 (d, 1H), 3.79 (d, 1H), 3.70 (dd, 1H), 3.62 (dd, 1H), 3.58 (dd, 1H), 3.45 (dd, 1H), 2.91 (sept, 1H), 2.70-2.45 (m, 4H), 1.27-1.24 (m, 18H) ppm.

### Preparation of 4-Oxo-4-[6-(2,4,6-triisopropyl-benzenesulfonyl)-3,6-diaza-bicyclo[3.1.0]hex-3-yl]-butyric acid 2,5-dioxo-pyrrolidin-1-yl ester 29a

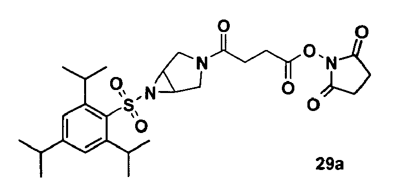

180 mg (0.4 mmol) **28a** were solved in 3.6 mL dichloromethane followed by the addition of 265 mg (0.6 mmol) BOP and 50.6 mg (0.44 mmol) N-Hydroxysuccinimide. The reaction mixture was cooled down to 0°C followed by the addition of 0.12 mL (0.72 mmol) diisopropyl ethyl amine. The mixture was stirred at room temperature over night, diluted with dichloromethane, washed with 10% citric acid, saturated aqueous bicarbonate solution and brine, dried over sodium sulphate, filtrated and concentrated. The residue was purified by chromatography on silica gel to give 85 mg (39%) of **29a**.
¹H-NMR (CDCl₃): δ = 7.17 (s, 2H), 4.27 (sept, 2H), 3.97 (d, 1H), 3.77 (d, 1H), 3.70 (dd, 1H), 3.62-3.58 (m, 2H), 3.42 (dd, 1H), 2.99-2.87 (m, 3H), 2.83 (s, 4H), 2.58 (m, 2H), 1.28-1.22 (div. d, 18H) ppm.

### Preparation of N-Benzyl-4-oxo-4-[6-(2,4,6-triisopropyl-benzenesulfonyl)-3,6-diaza-bicyclo[3.1.0]hex-3-yl]-butyramide 30aa

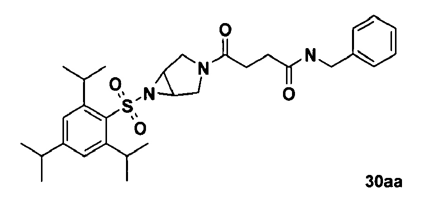

A: 96 mg (0.18 mmol) **29a** were solved in 2 mL DMF followed by the addition of 0.019 ml (0.18 mmol) benzyl amine. The reaction mixture was stirred over night at room temperature and concentrated. The residue was purified by chromatography on silica gel to give 31 mg (30%) of **30aa**.

B: 78 mg (0.17 mmol) **28a** were solved in 4 mL dichloromethane followed by the addition of 84.2 mg (0.19 mmol) BOP and 18.9 µL (0.17 mmol) benzyl amine. The mixture was cooled down to 0°C and 0.044 mL (0.26 mmol) diisopropyl ethyl amine was added. The reaction mixture was stirred over night at room temperature, diluted with dichloromethane, washed with washed with 10% citric acid, saturated aqueous bicarbonate solution and brine, dried over sodium sulphate, filtrated and concentrated. The residue was purified by chromatography on silica gel to give 68 mg (73%) of **30aa**.
¹H-NMR (CDCl₃): δ = 7.33-7.23 (m, 5H), 7.17 (s, 2H), 6.31 (bt, 1H), 4.39 (d, 2H), 4.27 (sept, 2H), 3.90 (d, 1H), 3.77 (d, 1H), 3.67 (dd, 1H), 3.63-3.57 (m, 2H), 3.36 (dd, 1H), 2.91 (sept, 1H), 2.61-2.43 (m, 4H), 1.28-1.21 (div. d, 18H) ppm.

### Preparation of 4-Oxo-4-[6-(2,4,6-triisopropyl-benzenesulfonyl)-3,6-diaza-bicyclo[3.1.0]hex-3-yl]-butyric acid-βAla-Phe-amide 30ab

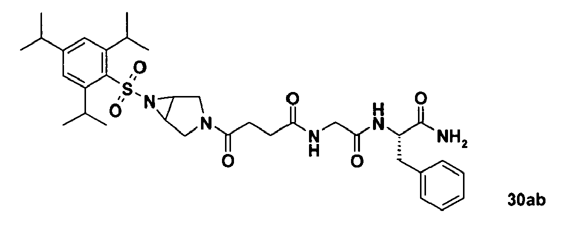

30 mg (0.067 mmol) **28a** were solved in 1 mL dichloromethane and 0.2 ml DMF followed by the addition of 10.42 µL (0.067 mmol) DIC and 18.7 mg (0.067 mmol) dipeptide. The reaction mixture was stirred over night at room temperature and concentrated. The residue was purified by chromatography on silica gel to give 21 mg (48%) of **30ab.**
MS (ES+): *m*/*z* (%) = 654 (100).

### Preparation of 4-Oxo-4-[6-(2,4,6-triisopropyl-benzenesulfonyl)-3,6-diaza-bicyclo[3.1.0]hex-3-yl]-butyric acid 3-(3-Amino-propyl)-1-[(2R,4S,SR)-4-(1-methoxy-cyclohexyloxy)-5-(1-methoxy-cyclohexyloxymethyl)-tetrahydro-furan-2-yl]-5-methyl-1H-pyrimidine-2,4-dione 30ac

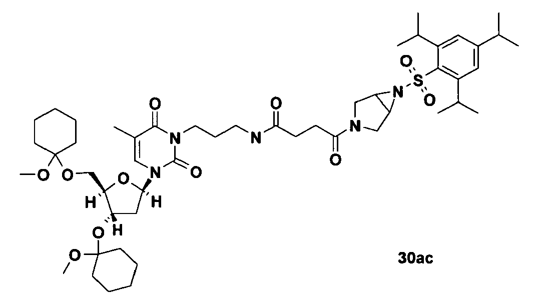

50 mg (0.11 mmol) **28a** were solved in 1.5 mL dichloromethane followed by the addition of 26.1 µL (0.17 mmol) DIC. After 30 min 58.1 mg (0.11 mmol) 3-(3-Amino-propyl)-1-[(2R,4S,5R)-4-(1-methoxy-cyclohexyloxy)-5-(1-methoxy-cyclohexyloxymethyl)-tetrahydro-furan-2-yl]-5-methyl-1H-pyrimidine-2,4-dione solved in 1 mL dichloromethane were added. The reaction mixture was stirred at room temperature over night, concentrated and the residue was purified by chromatography on silica gel to give 61 mg (57%) of **30ac.**
¹H-NMR (MeOD): δ = 7.68 (s, 1H), 7.30 (s, 2H), 6.32 (t, 1H), 4.60 (m, 1H), 4.37 (sept, 2H), 4.19 (dd, 1H), 3.98 (t, 2H), 3.89 (d, 1H), 3.85-3.68 (m, 5H), 3.64 (dd, 2H), 3.43 (d, 1H), 3.24 (s, 6H), 3.20 (t, 2H), 2.97 (sept, 1 H), 2.59-2.23 (m, 6H), 1.95 (s, 3H), 1.84-1.44 (m, 23H), 1.30-1.24 (div. d, 18H) ppm.

### Fluorination

### Preparation of N-Benzyl-4-[3-fluoro-4-(2,4,6-triisopropyl-benzenesulfonylamino)-pyrrolidin-1-yl]-4-oxo-butyramide 35aa

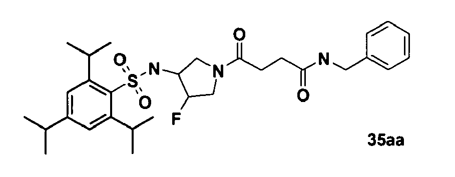

12 mg (0.022 mmol) **30aa** were solved in 0.7 mL DMSO followed by the addition of 1.42 mg (0.024 mmol) KF and 9.21 mg (0.024 mmol) Kryptofix K222. The reaction mixture was stirred at 50°C for 1h, diluted with saturated aqueous ammonium chloride solution and extracted with ethyl acetate. The combined organic phases were washed with brine, dried over sodium sulphate, filtrated and concentrated. The residue was purified by chromatography on silica gel to give 6 mg (48%) of **35aa**.
MS (ESI+): *m*/*z* (%) = 560 (100).

### Preparation of N-[((S)-1-Carbamoyl-2-phenyl-ethylcarbamoyl)-methyl]-4-[(3S,4S)-3-fluoro-4-(2,4,6-triisopropyl-benzene-sulfonylamino)-pyrrolidin-1-yl]-4-oxo-butyramide 35ab

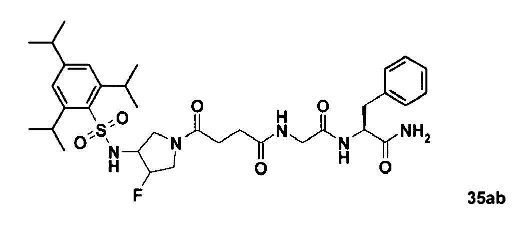

17 mg (0.026 mmol) **30ab** were solved in 0.8 mL DMSO followed by the addition of 1.66 mg (0.029 mmol) KF and 10.77 mg (0.029 mmol) Kryptofix K222. The reaction mixture was stirred at 50°C for 3h, diluted with saturated aqueous ammonium chloride solution and extracted with ethyl acetate. The combined organic phases were washed with brine, dried over sodium sulphate, filtrated and concentrated. The residue was purified by chromatography on silica gel to give 7.8 mg (44.5 %) of **35ab**.
MS (ESI+): m/z (%) = 674 (100).

### Preparation of 3-(3-Amino-propyl)-1-[(2R,4S,SR)-4-(1-methoxy-cyclohexyloxy)-5-(1-methoxy-cyclohexyloxymethyl)-tetrahydro-furan-2-yl]-5-methyl-1H-pyrimidine-2,4-dione 4-[3-fluoro-4-(2,4,6-triisopropyl-benzenesulfonylamino)-pyrrolidin-1-yl]-4-oxo-butyramide 35ac

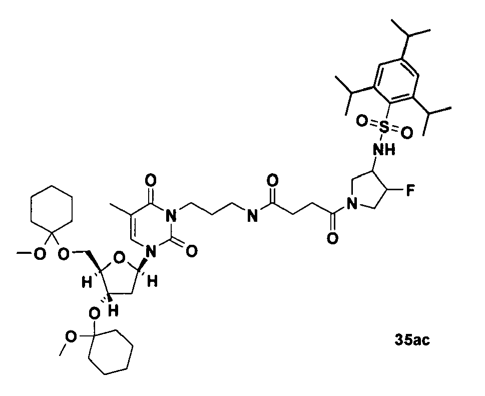

20 mg (0.021 mmol) **30ac** were solved in 0.7 mL DMSO followed by the addition of 1.34 mg (0.023 mmol) KF and 8.66 mg (0.023 mmol) Kryptofix K222. The reaction mixture was stirred at 50°C for 3h, diluted with saturated aqueous ammonium chloride solution and extracted with ethyl acetate. The combined organic phases were washed with brine, dried over sodium sulphate, filtrated and concentrated. The residue was purified by chromatography on silica gel to give 5.6 mg (27 %) of **35ac**.
MS (ESI+): *m*/*z* (%) = 977 (100).

### Radiochemistry

### General Radiolabelling Method

¹⁸F-Fluoride was azeotropically dried in the presence of Kryptofix 222 (5 mg in 1 ml MeCN) and potassium carbonate (1 mg in 0.5 ml water) by heating under nitrogen at 100-120°C for 20-30 minutes. During this time 3 × 1 ml MeCN were added and evaporated to give the dried Kryptofix 222/K₂CO₃ complex (up to 9.9 GBq). After drying, a solution of the precursor (200 µl of 22.6-30 mM in DMSO) was added. The reaction vessel was sealed and heated in the range of RT-90°C for 15 min to effect labeling. The crude reaction mixture was analyzed by analytical HPLC.

### Example A. Radiosynthesis of 3-[¹⁸F]Fluoro-N-benzyl-2-(4-methylphenylsulphonamido)-propanamide 11aa-18F

[¹⁸F]Fluoride was eluted from the QMA Light cartridge (Waters) into a Reactivial (10 ml) with a solution of Kryptofix 222 (5 mg), potassium carbonate (1 mg) in water (500 µl) and MeCN (1 ml). The solvent was removed by heating at 100°C under vacuum for 10 min with a stream of nitrogen. Anhydrous MeCN (1 ml) was added and evaporated as before. This step was repeated again to give the dried Kryptofix 222/K₂CO₃ complex (2.34 GBq). A solution of *N*-benzyl-1-tosylaziridine-2-carboxamide **10aa** (2 mg) in anhydrous DMSO (200 µl) was added. After heating at 70°C for 15 min, the reaction was cooled to room temperature and diluted with MeCN (1 ml). The crude reaction mixture was analyzed using an analytical HPLC (Column Nucleosil C18, 250x4 mm, 5 Å, 1 ml/min, solvent 10-40% MeCN in water gradient 15 min). >90 % conversion was observed.

### Example B. Radiosynthesis of -3-[¹⁸F]Fluoro-N-benzyl-2-(2,4,6-triisopropylphenylsulphon amido) -propanamide 11ab-18F

[¹⁸F]Fluoride was eluted from the QMA Light cartridge (Waters) into a Reactivial (10 ml) with a solution of Kryptofix 222 (5 mg), potassium carbonate (1 mg) in water (500 µl) and MeCN (1 ml). The solvent was removed by heating at 100°C under vacuum for 10 min with a stream of nitrogen. Anhydrous MeCN (1 ml) was added and evaporated as before. This step was repeated again to give the dried Kryptofix 222/K₂CO₃ complex (5.9 GBq). A solution of N-benzyl-1-(2,4,6-triisopropylphenylsulphonyl)-aziridine-2-carboxamide **10ab** (2 mg) in anhydrous DMSO (200 µl) was added. After heating at 60°C for 15 min, the reaction was cooled to room temperature and dilute with MeCN (1 ml). The crude reaction mixture was analyzed using an analytical HPLC (Column Nucleosil C18, 250x4 mm, 5 µÅ, 1 ml/min, solvent 40-95% MeCN in water gradient 20 min). >90 % conversion was observed.

### Example C. Radiosynthesis of 3-[¹⁸F]Fluoro-N-benzyl-2-(3,4-dimethoxyphenylsulphon-amido)-propanamide 11ac-18F

[¹⁸F]Fluoride was eluted from the QMA Light cartridge (Waters) into a Reactivial (10 ml) with a solution of Kryptofix 222 (5 mg), potassium carbonate (1 mg) in water (500 µl) and MeCN (1 ml). The solvent was removed by heating at 100°C under vacuum for 10 min with a stream of nitrogen. Anhydrous MeCN (1 ml) was added and evaporated as before. This step was repeated again to give the dried Kryptofix 222/K₂CO₃ complex (9.9 GBq). A solution of *N*-benzyl-1-(3,4-dimethoxyphenylsulphonyl)-aziridine-2-carboxamide **10ac** (2 mg) in anhydrous DMSO (200 µl) was added. After heating at 90°C for 15 min, the reaction was cooled to room temperature and dilute with MeCN (1 ml). The crude reaction mixture was analyzed using an analytical HPLC (Column Nucleosil C18, 250×4 mm, 5 µÅ, 1 ml/min, solvent 10-60% MeCN in water gradient 15 min). >90 % conversion was observed.

### Example D. Radiosynthesis of N-Benzyl-4-[3-[¹⁸F]fluoro-4-(2,4,6-triisopropyl-benzene-sulfonylamino)-pyrrolidin-1-yl]-4-oxo-butyramide 35aa-18F

0.6mL H¹⁸F/H₂¹⁸O eluted through a QMA Light cartridge (Waters) which was preconditioned with 5mL 0.5M K₂CO₃, followed by 5mL H₂O for trapping F18. [¹⁸F]Fluoride was washed down into a Reactivial (10ml) with a solution of Kryptofix 222 (5mg), potassium carbonate (1mg) in water (500µl) and MeCN (1.5ml). The solution was dried by heating at 105°C under vacuum and with a stream of nitrogen. Anhydrous MeCN (2 x 1ml) was added two times and evaporated for getting rid of water. A solution of N-Benzyl-4-oxo-4-[6-(2,4,6-triisopropylbenzenesulfonyl)-3,6-diaza-bicyclo[3.1.0]hex-3-yl]-butyramide **30aa** (2mg, 3.7µmol) in anhydrous DMSO (200µl) was added. After heating at 90°C for 15 min, the reaction was cooled to room temperature and dilute with MeCN (1ml). The crude reaction mixture was analyzed using an analytical HPLC Column (Lichrosorb RP18, 250 x 4 mm, 5µ, 1mL/min). F18 incorporation to the target compound was 97% with HPLC analytical method and 92% with TLC analysis.

### Hydrolytic stability of the β-fluoro amines

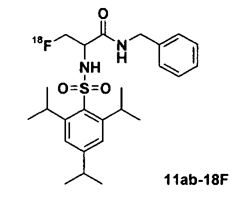

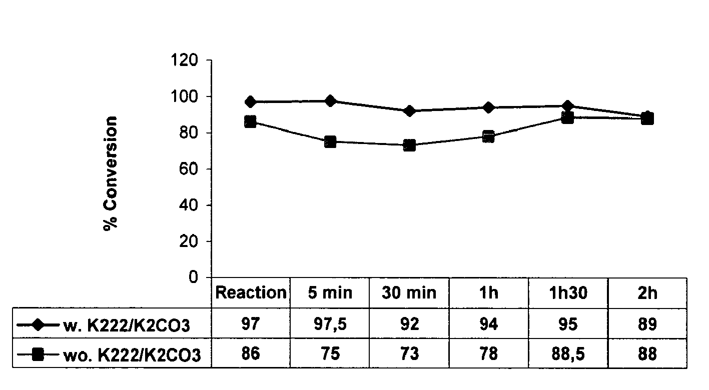

The β-fluoro amino acid derivative 11ab-18F is quite stable under neutral and basic conditions.

### Plasma stability of the β-fluoro amines

675 µL EtOH were added to the reactive-vial and then 5 aliquots of 70 µL of the Plasma solution were incubated for different time periods.

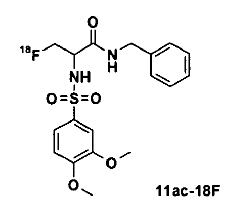

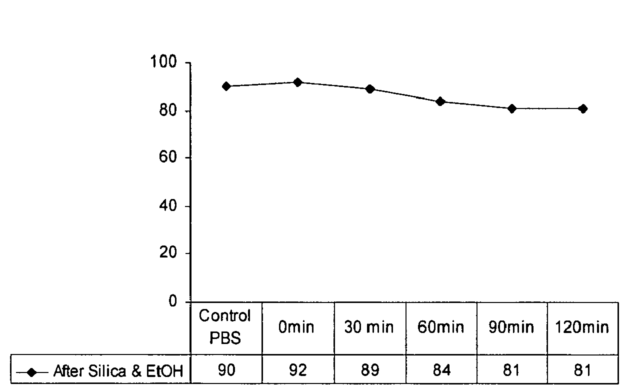

1 lac-18F is stable in solution with Human Plasma.

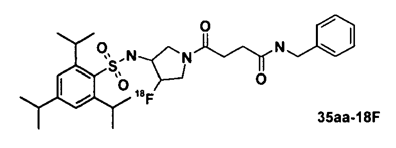

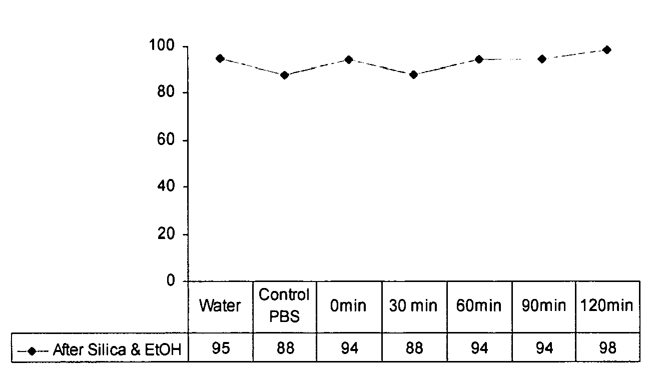

35aa-18F is stable in solution with Human Plasma.

## Claims

1. Compound comprising an for labelling purposes appropriately activated aziridine ring, wherein a targeting agent radical, either directly or via an appropriate linker, is attached either to the aziridine ring or to a fused 5-member carbocyclic or heterocyclic ring.

2. Compound according to claim 1 having formula I 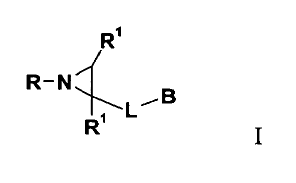 wherein
R represents Ts, 2,4,6-triisopropyl-phenyl-sulfonyl, 3,4-dimethoxy-phenyl-sulfonyl, phenyl-sulfonyl, with 1-5 R² substituted phenyl-sulfonyl, Ns, Cbz, Bz, Bn, Boc, Fmoc, methoxycarbonyl, ethoxycarbonyl, allyloxycarbonyl, Tr, or acyl;
R¹ represents hydrogen, substituted or non-substituted, linear or branched C₁-C₆ alkyl, cycloalkyl, heterocycloalkyl, aryl, heteroaryl, aralkyl, or heteroaralkyl;
R² represents hydrogen, substituted or non-substituted, linear or branched C₁-C₆ alkyl, cycloalkyl, heterocycloalkyl, aryl, heteroaryl, aralkyl, or heteroaralkyl, OH, OR³, NH₂, NHR³, NR³₂, SH, SR³, halogens, nitro, COR³, COOR³, CONHR³, CONR³₂;
R³ represents hydrogen, substituted or non-substituted, linear or branched C₁-C₆ alkyl, aryl, cycloalkyl, heterocycloalkyl, aryl, heteroaryl, aralkyl, or heteroaralkyl;
L represents a linker suitable for coupling with the targeting agent radical; and

3. Compound according to claim 1 having formula II 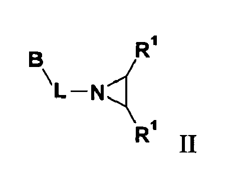 wherein
R¹, independently, represents hydrogen, substituted or non-substituted, linear or branched C₁-C₆ alkyl, cycloalkyl, heterocycloalkyl, aryl, heteroaryl, aralkyl, or heteroaralkyl;
L represents a linker suitable for coupling with the targeting agent radical and appropriate activation of the aziridine; and
B represents the targeting agent radical.

4. Compound according to claim 1 having formula III 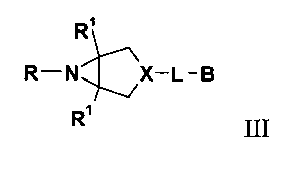 wherein
R represents Ts, 2,4,6-triisopropyl-phenyl-sulfonyl, 3,4-dimethoxy-phenyl-sulfonyl, phenyl-sulfonyl, with 1-5 R² substituted phenyl-sulfonyl, Ns, Cbz, Bz, Bn, Boc, Fmoc, methoxycarbonyl, ethoxycarbonyl, allyloxycarbonyl, Tr, or acyl;
R¹ represents hydrogen, substituted or non-substituted, linear or branched C₁-C₆ alkyl, cycloalkyl, heterocycloalkyl, aryl, heteroaryl, aralkyl, or heteroaralkyl;
R² represents hydrogen, substituted or non-substituted, linear or branched C₁-C₆ alkyl, cycloalkyl, heterocycloalkyl, aryl, heteroaryl, aralkyl, or heteroaralkyl, OH, OR³, NH₂, NHR³, NR³₂, SH, SR³, halogens, nitro, COR³, COOR³, CONHR³, CONR³₂;
R³ represents hydrogen, substituted or non-substituted, linear or branched C₁-C₆ alkyl, aryl, cycloalkyl, heterocycloalkyl, aryl, heteroaryl, aralkyl, or heteroaralkyl;
X represents N or C substituted by a hydrogen;
L represents a linker suitable for coupling with the targeting agent radical; and
B represents the targeting agent radical.

5. Compound according to any of claims 1 to 4, wherein the linker L is selected from the group consisting of substituted or non-substituted, linear or branched C₁-C₆ alkyl, cycloalkyl, alkenyl, heterocycloalkyl, aryl, heteroaryl, substituted aryl or heteroaryl, aralkyl, heteroaralkyl, alkoxy, aryloxy, aralkoxy, directly bound to an alkyl chain or aryl or - CO-, -C(=O)O-, -C(=O)NH-, -SO₂-, -SO₂NR³-, -NR³SO₂-, -NR³C(=O)O-, -NR³C(=O)NR³-, -NR³-, -NH-NH-, -NH-O- 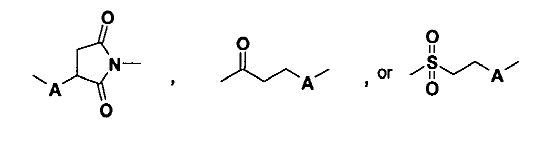 wherein A represents S or N; and
R³ represents hydrogen substituted or non-substituted, linear or branched C₁-C₆ alkyl, cycloalkyl, heterocycloalkyl, aryl, heteroaryl, aralkyl or heteroaralkyl.

6. Compound according to any of claims 1 to 5, wherein the targeting agent is selected from peptides, small molecules or oligonucleotides.

7. Compound according to any of claims 1 to 5, wherein the targeting agent is -NR⁴-peptide, R⁴ being independently selected from hydrogen or alkyl, or -(CH₂)ₙ- peptide, and wherein n is between 1 and 6.

8. Compound according to claim 6 or 7, wherein the targeting agent is a peptide comprising from 4 to 100 amino acids.

9. Method of preparing a compound according to any of claims 1 to 8 by reacting a suitable precursor molecule with the targeting agent or a precursor thereof.

10. Compound obtainable by a ring opening halogenation reaction of the aziridine ring of a compound according to any of claims 1 to 8, or a pharmaceutically acceptable salt or solvate thereof.

11. Compound according to claim 10, wherein the halogen introduced is ¹⁸F, ¹⁹F, ⁷⁵Br, ⁷⁶Br, ¹²³I, ¹³¹I or ^{34m}Cl.

12. Compound according to claim 11, wherein the halogen introduced is ¹⁸F.

13. Method of preparing a compound according to any of claims 10 to 12 by reacting a compound according to any of claims 1 to 8 with an appropriate halogenation agent under appropriate reaction conditions.

14. Method according to claim 13, wherein said halogenation agent is K¹⁸F, H¹⁸F, KH¹⁸F₂ or a tetraalkyl ammonium salt of ¹⁸F⁻.

15. Method according to claim 14, wherein the reaction temperature is 100°C or less.

16. Method according to claim 15, wherein the reaction temperature is 80°C or less.

17. Composition comprising a compound according to any of claims 10 to 12.

18. Composition according to claim 17, further comprising a physiologically acceptable diluent or carrier.

19. Kit comprising a compound of any of claims 10 to 12 or a composition of claim 17 or 18, in powder form, and a container containing an appropriate solvent for preparing a solution of the compound or composition for administration to an animal, including a human.

20. Use of a compound according to any of claims 10 to 12, composition of claim 17 or 18, or kit of claim 19 for diagnostic imaging.

21. Use according to claim 20 for positron emission tomography.

22. Use according to claim 20 or 21 for imaging of tumors, imaging of inflammatory and/or neurodegenerative diseases, such as multiple sclerosis or Alzheimer's disease, or imaging of angiogenesis-associated diseases, such as growth of solid tumors, and rheumatoid arthritis.
